# EUROPEAN PATENT SPECIFICATION

(11) **EP 3 253 381 B1**
(45) Date of publication and mention of the grant of the patent: **11.11.2020**
(21) Application number: 16706474.0
(22) Date of filing: 05.02.2016
(51) Int. Cl.: A61K 31/14, A61K 31/205, A61P 9/10

(54) **TRIMETHYLAMINE-N-OXIDE PRODUCING AGENT FOR TREATING ATHEROMA FORMATION**
TRIMETHYL-N-OXIDE-ERZEUGENDES MITTEL ZUR BEHANDLUNG DER ATHEROMA-BILDUNG
AGENT PRODUCTEUR DE TRIMETHYLAMINE-N-OXIDE POUR TRAITER LA FORMATION D'ATHÉROME

(30) Priority: 06.02.2015 US 201562112945 P
(43) Date of publication of application: 13.12.2017
(73) Proprietor: Lonza Consumer Health Inc., Benicia, CA 94510 (US)
(72) Inventor: BELLAMINE, Aouatef, Allendale, NJ 07401 (US); WILLIAMSON, Yuping Vivian, Allendale, NJ 07401 (US)
(74) Representative: Rummler, Felix
(86) International application number: PCT/US2016/016732
(87) International publication number: WO 2016/127034

(56) References cited:
- WO-A2-2007/111992
- AOUATEF BELLAMINE ET AL: "TMAO an L-carnitine metabolite does not affect foam cell formation or cholesterol efflux in J774 mouse macrophage (249.6)", THE FASEB JOURNAL, vol. 28, no. 1, Suppl., 1 April 2014 (2014-04-01), XP055267951, US ISSN: 0892-6638

## Description

### RELATED APPLICATIONS

The present application is based on and claims priority to U.S. Provisional Patent application Serial No. 62/112,945, filed on February 6, 2015.

### BACKGROUND

Heart and cardiovascular diseases continue to be the leading causes of death for both men and women. One cardiovascular disease is atherosclerosis. Atherosclerosis is characterized by the deposits or plaques of lipids and other blood derivatives in the arterial walls. Due to the disease, artery walls can thicken due primarily to the accumulation of fat engorged white blood cells. Atherosclerosis is typically associated with the hardening of the arteries. The disease can affect all arteries in the body, and particularly the aorta and the coronary arteries.

Atherosclerotic plaques begin as fatty streaks underlying the endothelium of large arteries, such as the aorta. The fatty streaks can contain both living cells and dead cells, including cholesterol and triglycerides. Other time, the fatty streaks or plaques can be calcified turning into calcium and other crystallized materials. The development of plaques, especially in the calcified form, can reduce the elasticity of the artery walls. Wall stiffening can eventually increase blood pressure and ultimately decrease blood flow. When plaques rupture, they can lead to vascular obstruction, ischemia and cardiac event.

The causes of atherosclerosis are not completely known. It is believed that inflammation and high levels of certain cholesterol in the blood can lead to atherosclerosis. Specifically, high blood levels of low density lipoprotein (LDL)) cholesterol particles, which are microscopic particles consisting of an outer rim of lipoprotein surrounding a cholesterol center, increase the likelihood that atherosclerosis will progress. It is believed that the LDL particles deposit in the walls of arteries when the endothelial cell layer function is compromised. LDL particles also cross the endothelial cells into the inner layers of the blood vessels, and under the effect of inflammatory factors, LDL can be oxidized to ox-LDL. When the oxidized form appears, more white blood cells migrate to the area and penetrate into the arterial walls. These white blood cells can then transform into macrophages.

The formation of macrophages may create an immune response in the body. More white blood cells are brought to the area and absorb the oxidized LDL particle forming foam cells. As further foam cells form, the artery can become constricted. In some instances, the foam cells die and form the fatty streak and the plaques. These eventually rupture which can lead to ischemia and cardiac event.

In the past, various different methods have been proposed in order to treat and prevent atherosclerosis and medical conditions that are associated with or mediated by atherosclerosis. For instance, one common way to prevent atherosclerosis is to change one's diet. Those skilled in the art have suggested a diet that is low in LDL-forming cholesterol.

In addition to changing one's diet, various other pharmaceutical products have been suggested. Most of these products target LDL and cholesterol lowering. For instance, statins have been administered to patients for lowering LDL cholesterol. Another group of drugs known as fibrates are used to reduce triglyceride levels. Other suggested drugs include niacin, zetia, bile acid sequestrants and plant sterols. Most of these drugs are directed to lowering cholesterol levels in the blood.

CETP (cholesteryl ester transport protein is the enzyme that transforms HDL (high density lipoprotein) or "good cholesterol" to the pro-atherogenic LDL. This enzyme has been also the target for new drug development. Its inhibition leads to higher HDL levels and lower LDL levels.

Atherosclerosis, however, still continues to be a significant health threat. Thus, a recognized need exists for better and more reliable means for treating and preventing or slowing down the atherosclerosis disease progression. In particular, a need exists for newer pharmaceutical products with different mechanism of actions than lowering cholesterol in treating, reducing, preventing and/or inhibiting athero lesions from forming.

WO 2007/111992 A2 relates to the use of TMAO or TMAO derivatives in the treatment or prevention of artherosclerosis.

### SUMMARY

The present invention relates to a TMAO producing agent for use in a method for treating artherio lesions comprising: administering to a mammal a TMAO producing agent, the TMAO producing agent comprising a compound that, when ingested or injected, forms a TMAO in the body of the mammal, the TMAO producing agent being administered to the mammal in a pharmaceutically effective amount to increase TMAO levels in the blood of the mammal, wherein the TMAO producing agent comprises a carnitine, a choline, a betaine, or combinations thereof, wherein the carnitine comprises L-carnitine or D-carnitine, and the betaine comprises a betaine salt, a carboxylic derivative of betaine, or a betaine/amino acid complex or compound, or combinations thereof. The invention is defined in claim 1 or any one of its dependent claims which latter define particular invention embodiments.

Where "disclosure" is mentioned, this relates to subject matter disclosed for reference purposes. Where "according to the invention" or "in accordance with the present invention" is mentioned, this relates to an embodiment falling under claim 1 or a dependent claims ("claims"). In general, the present disclosure is directed to methods for treating atherosclerosis. The present disclosure is also directed to a system or kit for treating atherosclerosis. The method, system or kit of the present disclosure can also be used to treat other disorders associated with or mediated by atherosclerosis or at risk of developing atherosclerosis.

In one embodiment, the present disclosure is directed to a method for treating athero lesions that may develop as a result of atherosclerosis or a related disease. The method includes the step of administering to a mammal, and particularly a human patient, a TMAO producing agent. The TMAO producing agent comprises a compound that comprises or chemically converts into trimethylamine and/or trimethylamine N-oxide then to TMAO when ingested or injected. The TMAO producing agent is administered to the mammal in a pharmaceutically effective amount sufficient to increase trimethylamine N-oxide levels in the blood of the mammal. A potential TMAO-like molecule can also be ingested or injected directly and may show similar effects.

The present inventor discovered that increasing trimethylamine N-oxide levels in the body can significantly and dramatically reduce the appearance of athero lesions.

In accordance with the present invention as defined in the claims, the TMAO producing agent comprises D-carnitine or L-carnitine. In an alternative embodiment, the TMAO producing agent comprises a choline. In still another embodiment, the TMAO producing agent comprises a betaine. Betaines that may be used include betaine salts, carboxylic derivatives of betaine, and/or betaine/amino acids. In still another embodiment, the TMAO producing agent may comprise a combination of the TMAO producing agents. For instance, the composition may comprise a mixture of at least two TMAO producing agents selected from the group consisting of a carnitine, a choline, and a betaine.

In one disclosure embodiment, during administration of the TMAO producing agent to the mammal, the levels of trimethylamine N-oxide are monitored in the blood of the mammal. Based on the monitored levels, the amount of the TMAO producing agent administered to the mammal may be increased. For exemplary purposes only, in one embodiment the TMAO producing agent may be administered to the mammal so that, depending on mammal species and individual patient, TMAO levels in the blood are greater than 0.1 ppm, such as greater than 0.12 ppm, such as greater than 0.15 ppm.

The dosage of the TMAO producing agent can vary depending upon numerous factors including the mammal being treated and the condition of the patient. In one embodiment, each dose can be greater than 150 mg/kg, such as greater than 200 mg/kg, such as greater than 250 mg/kg, such as greater than 300 mg/kg, such as greater than 350 mg/kg, such as greater than 400 mg/kg of a TMAO producing agent. In another embodiment, relatively small amounts of the TMAO producing agent are administered to the mammal. For instance, each dose can be from 7 to 150 mg/kg, such as from 40 to 150 mg/kg, such as greater than 50 mg/kg, such as greater than 70 mg/kg, such as greater than 100 mg/kg of a TMAO producing agent. The dose is generally less than 1,000 mg/kg, such as less than 900 mg/kg, such as less than 800 mg/kg, such as less than 700 mg/kg, such as less than 600 mg/kg of a TMAO producing agent. The patient can be fed a dose showing an efficacy during the development of the compound periodically over an extended period of time. In one embodiment, the mammal can be administered one to two doses a day.

In one embodiment according to the invention, the mammal or patient treated in accordance with the present disclosure is a patient afflicted with or at risk of developing atherosclerosis or a disorder associated with or mediated by atherosclerosis. For instance, the patient may have a peripheral vascular disease.

The present disclosure is also directed to a system or kit for treating or preventing atherosclerosis. The system or kit includes a TMAO producing agent in conjunction with an article that facilitates monitoring trimethylamine N-oxide levels in a blood sample taken from the mammal or patient. The article, for instance, may include educational and/or instructional material for determining or analyzing the levels of trimethylamine N-oxide in the blood. The article, for instance, may comprise any device that facilitates taking a blood sample from the patient. In still another disclosure embodiment, the article may comprise an assay device for determining trimethylamine N-oxide levels.

The present disclosure is also directed to a method for determining analyte levels in plasma samples, wherein the analyte is TMAO, a TMAO precursor, or a TMAO analog. In one disclosure embodiment, the process includes the steps of producing at least two working standard mixtures comprising known but varied concentrations of the unlabeled analyte and known, fixed concentrations of the isotope-labeled analyte; analyzing said working standard mixtures using high performance liquid chromatography - mass spectroscopy (LC-MS)with multiple reaction monitoring (MRM); creating a reference or correlation for indicating the concentration of an non-labeled analyte based upon a comparison of analysis of the response of non-labeled analyte to the isotope-labeled analyte; producing at least one plasma sample solution comprising plasma sample, water, and a known, fixed concentration of the isotope-labeled analyte; producing at least one spiked plasma sample comprising a plasma blank, a known, varied concentration of the unlabeled analyte, and a known, fixed concentration of the isotope-labeled analyte; analyzing said plasma sample solutions using high performance liquid chromatography - mass spectroscopy with multiple reaction monitoring; and determining the concentration of said analyte in said plasma sample solution by comparing to said reference the response of the non-labeled analyte in plasma sample to the isotope-labeled analyte.

For instance, in one disclosure embodiment you can create a standard curve using the two or more working standard mixtures for use in determining the concentration of an analyte in a plasma sample solution. In another embodiment, a spiked plasma sample is prepared for each corresponding working standard.

Other features and aspects of the present disclosure or, as far as they fall under the claims, of the invention are discussed in greater detail below.

### BRIEF DESCRIPTION OF THE DRAWINGS

A full and enabling disclosure of the present disclosure or, as far as it falls under the claims, according to the invention is set forth more particularly in the remainder of the specification, including reference to the accompanying figures, in which:
Figure 1 is a diagram illustrating the location of the aortic root and en-face aorta for use in conjunction with the examples below; and
Figures 2-7 are graphical representations of the results obtained in the examples below; and

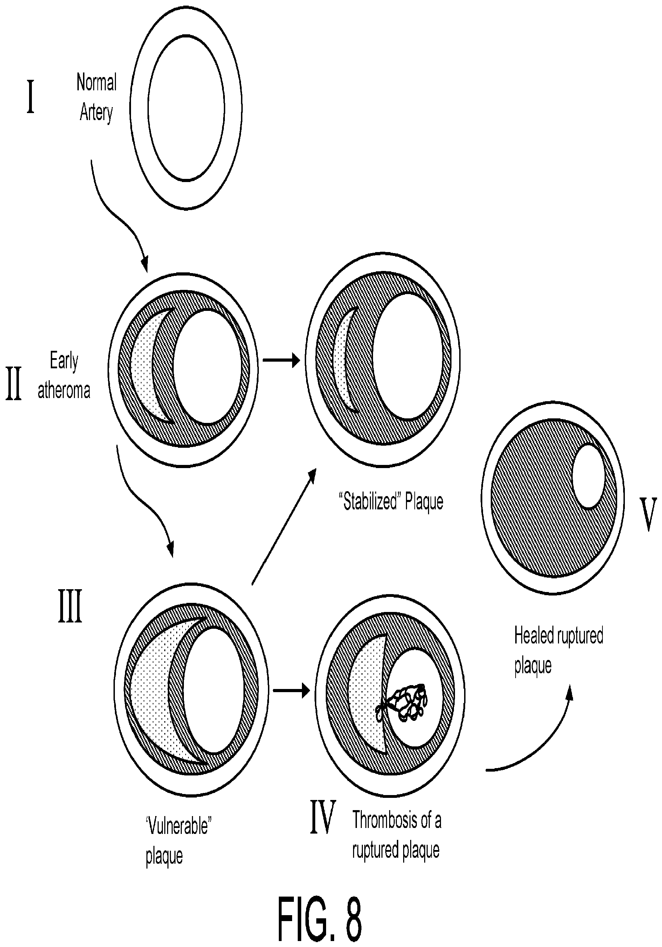

Figure 8 is a diagram illustrating the stages of atherosclerosis progression for use in conjunction with the examples below.

### DEFINITIONS

As used herein, atherosclerosis refers to a condition characterized by the hardening and/or narrowing of the arteries caused by the buildup of atheromatous plaque inside the arterial walls. The atheromatous plaque is divided in three components, (1) the atheroma, a nodular accumulation of a soft flaky material at the center of large plaques, composed of macrophages nearest the lumen of the artery; (2) underlying areas of cholesterol crystals; (3) calcification at the outer base of more advanced lesions. Indicators of atherosclerosis include, for example, the development of plaques in the arteries, their calcification, the extent of which can be determined by Sudan IV staining, or the development of foam cells in arteries. The narrowing of the arteries can be determined by coronary angioplasty, ultrafast CT, or ultrasound. In order to diagnose atherosclerosis disease one can look for atherosclerosis disease biomarkers as indicators such LDL-c, HDL-c CRP and others.

A pharmaceutically effective amount or therapeutically effective amount means an amount of a compound that treats, attenuates, ameliorates, eliminates, or prevents the particular condition or disorder or the particular symptom of the condition or disorder.

As used herein, for reference disclosure purposes a TMAO producing agent refers TMAO itself, TMAO analogs, TMAO precursors, TMAO analogs, TMAO-like molecules, TMA and any compound capable of converting into trimethylamine and/or into trimethylamine N-oxide by bacteria within the digestive tract alone or in conjunction with enzymes, such as liver enzymes.

As used herein, a carnitine, a choline, or a betaine includes derivatives and salts thereof. For instance, a carnitine includes D-carnitine and/or L-carnitine and variations thereof including, for example, L-carnitine base, L-carnitine HCL, L-carnitine L-tartrate, L-carnitine fumarate, proprinial L-carnitine, GPLC glycine proprinale L-carnitine, other carnitine derivatives and the like.

As used herein, a mammal includes any mammal that may experience atherosclerosis or an atherosclerosis-type disease or illness and includes canine, feline, equine, ovine, human, porcine and other mammals.

### DETAILED DESCRIPTION

In general, the present disclosure is directed to a method for treating or preventing atherosclerosis. The present disclosure is also directed to treating or preventing disorders mediated by or associated with atherosclerosis. In one embodiment, the method of the present disclosure or the TMAO producing agent for use according to the invention is directed to treating athero lesions that may result from atherosclerosis.

In order to treat or prevent atherosclerosis and/or (as invention embodiment according to the invention as far as it is defined by the claims) prevent athero lesions in accordance with the present disclosure, a TMAO producing agent is administered to a mammal in a pharmaceutically effective amount that increases trimethylamine N-oxide levels in the blood of the mammal. The TMAO producing agent (if defined by the claims according to the invention) is a compound that comprises or chemically converts to trimethylamine N-oxide in the body. For instance, in one embodiment, a TMAO producing agent is used that produces timethylamine in the digestive tract due to the presence of digestive bacteria. The timethylamine is then absorbed into the blood and converted into trimethylamine N-oxide in the liver. For instance, in the liver, trimethylamine may be contacted with one or more enzymes that produce trimethylamine N-oxide. The enzyme may comprise, for instance, a flavin containing mono-oxygenase (FMO). For instance, one of many enzymes, FMO3, is a liver enzyme located in the endoplasmic reticulum of the liver of humans.

Various different TMAO producing agents may be used in accordance with the present disclosure. For instance, the TMAO producing agent may comprise any suitable compound capable of increasing trimethylamine N-oxide levels in the body. Particular examples of TMAO producing agents for use according to the invention include a carnitine, a betaine, and/or choline. In one embodiment, a mixture of at least two TMAO producing agents may be used.

Examples of carnitines that may be used according to the invention include L-carnitine and/or D-carnitine. L-carnitine is a quaternary amine that can be biosynthesized from lysine and methionine. L-carnitine is known to promote beta-oxidation of long-chain fatty acids by facilitating a transfer across the mitochondrial membrane. D-carnitine, on the other hand, is not known to have the same oxidative effect. Both carnitines, however, are believed to be capable of increasing trimethylamine N-oxide levels and therefore both may be used in accordance with the present disclosure.

In the past, L-carnitine has been used as a nutritional supplement with other compounds for use as a lipid-lowering agent. For instance, PCT Publication WO03/009854 is directed to a lipid-lowering composition comprising carnitine and phytosterol. Carnitine as used in the present disclosure, however, is used to increase trimethylamine N-oxide levels and therefore operates under a different mechanism than that disclosed in the above publication.

In addition to carnitine, the TMAO producing agent according to the invention may also comprise a betaine. The betaine may comprise a betaine salt, a carboxylic derivative of betaine, and/or a betaine/amino acid complex or compound. Any suitable betaine may be used that is safe for consumption by mammals and that increases trimethylamine N-oxide levels in the mammal. Examples of betaines that may be used in accordance with the present disclosure or according to the invention include Trimethylglycine (standard Betaine), Betaine hydrochloride, Betaine dihydrogencitrate (Betaine citrate), Betaine monohydrate, Betaine sodium aspartate, Betaine-glycocyamine, Gamma-butyrobetaine, Proline-butane, Betaine glutamate, Cocamidylpropyl butane, or mixtures thereof.

In another embodiment of the present disclosure or according to the present invention, the TMAO producing agent comprises a choline compound. In general, any suitable choline compound can be used that is safe for consumption by a mammal and that is capable of increasing trimethylamine N-oxide levels in the mammal.

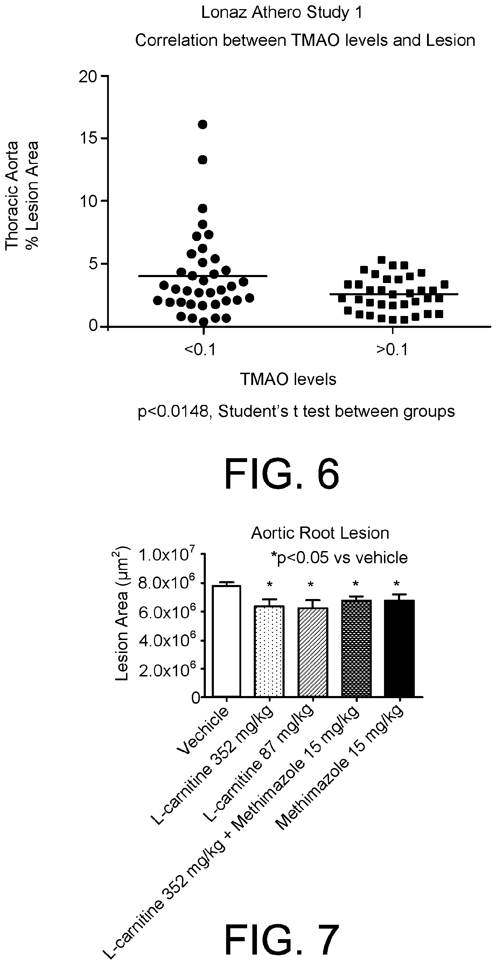

Figure 7 illustrates the progression of atherosclerosis through five stages. Stage I illustrates a normal artery without atherosclerotic plaques, while Stage II illustrates early atheroma, in which a soft flaky material forms a nodular accumulation at the center of large plaques, composed of lipid-engorged macrophages nearest the lumen of the artery. At this stage, the plaque possesses a lipid-rich core. While this early atheroma may develop in a 'stabilized' plaque, possessing a smaller lipid pool, a thick fibrous cap, and a preserved lumen, the atheroma may also develop into a 'vulnerable plaque'. As Stage III illustrates, in a 'vulnerable plaque' the lipid-rich core continues to grow, inflammatory cells accumulate, and the extracellular matrix begins to degrade, leading to the formation of a thin fibrous cap. If this thin fibrous cap is disturbed, the plaque may rupture, as shown in stage IV, and release thrombogenic material into the blood. This rupture can potentially lead to thrombus formation, which through extended occlusion of the blood vessel can result in an acute myocardial infarction. In Stage V, after this rupture, a wound healing response may occur, leading to a fibrous intima and a narrow lumen.

By increasing trimethylamine N-oxide levels in a mammal through the use of TMAO producing agents, atherosclerosis and other related diseases can be treated and/or prevented. It was discovered that the increase in trimethylamine N-oxide levels reduced athero lesions and particularly aortic lesions. Although unknown, it appears that trimethylamine N-oxide has a protective effect against atherosclerosis development. When lesions begin to develop, it appears that increased levels of trimethylamine N-oxide in the body acts as a compensatory mechanism. It is believed that increased consumption of TMAO producing agents do not have a significant impact on lipid content of aortic tissues and plasma levels and do not have a significant impact on foam cell formation. Consequently, although unknown, it is believed that the decrease in lesions as a result of increased levels of trimethylamine N-oxide takes place at a later stage in the atherosclerosis development. For instance, the increased trimethylamine N-oxide levels may serve as an agent in the regression of lesions.

This discovery is particularly surprising in view of what is believed to be a common misconception regarding the presence of trimethylamine N-oxide in the body. For instance, instead of treating or preventing atherosclerosis, those skilled in the art have indicated that trimethylamine N-oxide actually causes atherosclerosis to develop. For instance, an article entitled "Trimethylamine-N-Oxide, A Metabolite Associated With Atherosclerosis, Exhibits Complex Genetic and Dietary Regulation" by Bennett, et al. states that trimethylamine N-oxide is identified as a metabolite strongly associated with atherosclerosis. According to the article, trimethylamine N-oxide appears to contribute to the development of atherosclerosis in part by promoting cholesterol accumulation within macrophages, perhaps by inducing scavenger receptors which are involved in the uptake of modified lipoproteins by macrophages and contributing to foam cell formation. Other studies have also concluded that trimethylamine N-oxide promotes the development of heart disease, based on this association. Thus, the administration of TMAO producing agents to patients in order to treat or prevent atherosclerosis is an unexpected and surprising discovery.

In accordance with the present disclosure and as the agent for use according to the invention, the TMAO producing agent may be administered to any mammal in any suitable form using any suitable administration route. For example, the TMAO producing agent can be administered orally alone or in combination with a food composition. In one embodiment, the TMAO producing agent may be part of a dietary supplement, a food, a beverage, a nutraceutical composition, or as a medicament.

The TMAO producing agent can be administered to any mammal for treating atherosclerosis or a related disease. The TMAO producing agent may also be administered to mammals already suffering from atherosclerosis or any disorder associated with atherosclerosis. In particular, the TMAO producing agent for use according to the invention may be administered to any mammal that is subject to or may be undergoing the formation of athero lesions. The method of the present disclosure may be used to treat coronary disorders, vascular disorders, aneurysms, artery diseases, and the like.

According to the invention, the mammal may be canine, feline, or equine. The patient may also be bovine or porcine. In one embodiment, the patient is a human.

The amount of TMAO producing agent administered to a mammal in accordance with the present disclosure or to be administered according to the invention can vary depending upon different factors and circumstances. For instance, the amount can depend upon the diet and metabolism of the patient, the age of the patient, whether the patient is currently suffering from a vascular disease or atherosclerosis, and the like. In one embodiment, the TMAO producing agent for use according to the invention is administered to a mammal at a dose of from 40 mg/kg (mg TMAO producing agent per kg body weight) to 1,000 mg/kg. For instance, the TMAO producing agent may be administered to a mammal at a dose of greater than 40 mg/kg, such as greater than 50 mg/kg, such as greater than 60 mg/kg. For many patients, greater amounts may increase trimethylamine N-oxide levels to a desired level. For instance, in one embodiment, the TMAO producing agent for use according to the invention is administered to the mammal at a dose of greater than 150 mg/kg, such as greater than 170 mg/kg, such as greater than 200 mg/kg, such as greater than 220 mg/kg, such as greater than 250 mg/kg, such as greater than 300 mg/kg, such as greater than 350 mg/kg, such as greater than 400 mg/kg. In general, the TMAO producing agent is administered at an amount less than 1,000 mg/kg, such as less than 900 mg/kg, such as less than 800 mg/kg.

The TMAO producing agent for use according to the invention can be administered to the mammal at periodic intervals, such as once or twice a day. In other embodiments, the TMAO producing agent may be administered to the mammal at least one to three days, such as at least two to four times a week. In one particular embodiment, the TMAO producing agent is administered to a patient daily. For instance, the doses above may refer to doses per day even if the TMAO producing agent is administered more than once a day.

In one reference disclosure embodiment, trimethylamine N-oxide levels are monitored during periodic administration of the TMAO producing agent. In this manner, the amount of TMAO producing agent administered to the mammal can be adjusted based upon reaching desired trimethylamine N-oxide levels in the blood

As described above, the TMAO producing agent may be for use according to the invention in a method where it is administered to the mammal as a supplement and/or in conjunction with food compositions. In one embodiment, the TMAO producing agent for use according to the invention may be administered to a mammal in the form of a pharmaceutical composition that contains an acceptable carrier or excipient. For example, pharmaceutically acceptable carriers may comprise any composition that does not interfere with the ability of the TMAO producing agent to increase trimethylamine N-oxide levels in the body. Such carriers may include solvents, dispersion media, coatings, and the like. Particular materials that can be formulated with the TMAO producing agent include water, saline, polyalkylene glycols such as polyethylene glycol, vegetable oils, and the like. Other components that can be formulated with the TMAO producing agent include biodegradable polymers, such as lactides, or the like.

In general, the TMAO producing agent for use according to the invention is administered to a mammal orally. In general, however, the TMAO producing agent for use according to the invention may be administered to a mammal in any suitable form capable of increasing trimethylamine N-oxide levels. For example, the TMAO producing agent for use according to the invention may also be administered using other routes such as intranasal, intragastric, injection, and the like.

In one disclosure embodiment, the TMAO producing agent is provided to patients or caregivers in the form of a kit or system. For instance, the TMAO producing agent can be provided in conjunction with an article that facilitates monitoring trimethylamine N-oxide levels in a blood sample taken from a mammal. The article, for instance, may comprise informational or educational material used in determining or interpreting trimethylamine N-oxide levels in the blood. The article may also comprise a blood collection device. In still another embodiment, the article may comprise a trimethylamine N-oxide assay device.

The present disclosure may be better understood with reference to the following examples.

### EXAMPLES

The following examples are provided to further illustrate the invention but not to limit its scope. Other variants of the invention will be readily apparent to one of ordinary skill in the art and are encompassed by the appended claims.

### Example 1

The treatment effects of L-carnitine and the FMAO inhibitor methimazole were observed based on the on the serum lipid profile and progression of atherosclerosis in a mouse model of atherosclerosis. ApoE KO mice, male and 6 to 7 weeks of age, were transfected in vivo with AAV-CETP. Two weeks after infection, CETP expression in plasma was verified using a commercially available ELISA kit, and the mice were placed on a Western (high-fat) diet.

The mice were sorted into several groups based on matching initial total cholesterol and verified CETP expression: 1 - control group receiving a dose of dH₂O. 2 - a group receiving a high dose of L-carnitine (35.2 mg/mL dH₂O). 3 - a group receiving a low dose of L-carnitine (8.7mg/mL dH₂O). 4 - a group receiving a high dose of L-carnitine (35.2 mg/mL dH₂O) and a dose of methimazaole (1.5 mg/mL dH₂O). 5 - a group receiving a dose of methimazaole (1.5 mg/mL dH₂O).

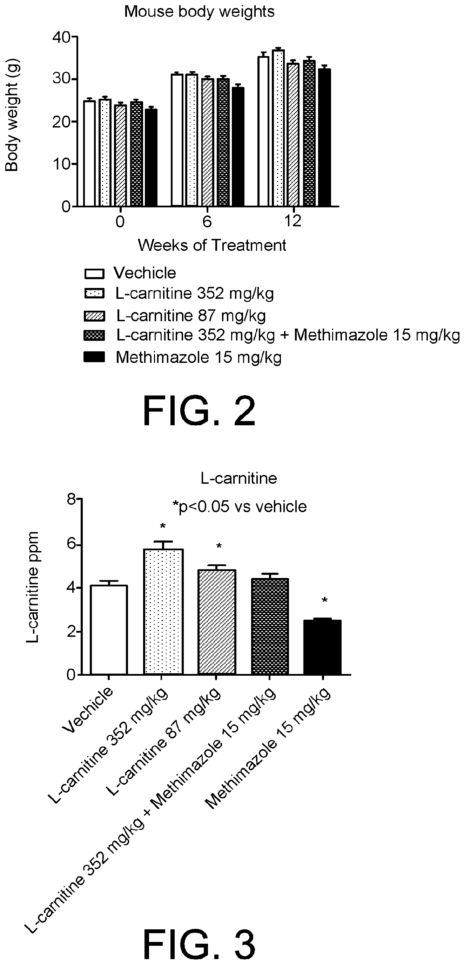

Dosing began when the mice reached 10 weeks of age and continued for 12 weeks. Drugs were administered once daily by oral gavage. The animals' body weights were measured weekly. No statistically significant changes in body weight were observed between treatment groups during the study (Figure 2).

The concentrations of L-carnitine and TMAO in the plasma were measured using a liquid chromatography - mass spectrometry method. A deuterium labeled L-carnitine stock standard was prepared using the weigh by difference technique. About 5 mg of d9-L-carnitine from Cambridge Isotope Laboratories were added to a 10 mL volumetric flask via a weighing funnel or weighing paper, and water:methanol = 1:1 was used to dissolve the sample.A deuterium labeled TMAO stock standard was prepared by accurately weighing 25 ± 2 mg of d9-trimethylamine N-oxide reference standard, transferring the sample into a 25 mL volumetric flask, adding about 15 mL of water: methanol = 1: 1, and vortexing until dissolved. The flask was then filled to volume with water: methanol = 1:1

Stock standards of deuterium labeled L-carnitine and deuterium labeled TMAO were prepared using 5 mg/10 mL water/methanol of d9-L-carnitine and 25 mg/25 mL water/methanol of d9-TMAO. These stock standards were used to prepare an internal standard by adding 200 µL of d9-L-carnitine stock standard and 10 µL of d9-TMAO into a 50 mL volumetric flask, adding acetonitrile to volume, and mixing well.

Calibration standards were then prepared. To make the L-carnitine stock standard, 25 ± 2 mg of L-carnitine reference standard was transferred to a 10 mL volumetric flask and approximately 7 mL of water: methanol = 1:1 were added. The solution was vortexed until dissolved and the filled to volume with water: methanol = 1:1. To make the trimethylamine N-oxide stock standard, 25 ± 2 mg of L-carnitine reference standard was transferred to a 25 mL volumetric flask and 15 mL of water:methanol = 1:1 were added. The solution was vortexed until dissolved and filled to volume with water:methanol = 1:1. These stock standards were then used to prepare an intermediate standard by pipetting 480 µL of L-carnitine stock standard and 100 µL of trimethylamine N-oxide into a 10 mL flask, adding water: methanol = 1:1 to volume, and mixing well.

Working standards were prepared according to Table 1 using water as diluent. For each standard, the designated amount of intermediate standard was pipetted into separate 10 mL volumetric flasks, and 7 mL of water were added. The solution was vortexed until dissolved and filled to volume with water.

**Table 1: Working Standard Concentrations**

| **L-carnitine (µg/mL)** | **Trimethylamine N-oxide (µg/mL)** | **Intermediate standard** | **Final volume (mL)** |
|---|---|---|---|
| 0.6 | 0.05 | 50 µL | 10 |
| 1.2 | 0.1 | 100 µL | 10 |
| 3 | 0.25 | 250 µL | 10 |
| 6 | 0.5 | 500 µL | 10 |
| 12 | 1 | 1.0 mL | 10 |

Working calibration standards were prepared by pipetting 2.5 mL of working standard solution, 2.5 mL of water, and 5 mL of internal standard solution into a flask and mixing well.

Plasma sample solutions were also prepared For the plasma sample solution, first 50 µL of mouse plasma sample and then 50 µL of water were added to a 2 mL centrifuge tube and mixed well. Next 100 µL of internal standard solution were added to the centrifuge tube and vortexed for 2 minutes after securing the cap. The mixture was then centrifuged. The plasma blank solution for spike recovery and the spiked plasma sample preparation were prepared similarly. For the plasma blank solution, 50 µL of mouse plasma blank and then 50 µL of water were pipetted into a 2 mL centrifuge tube and mixed well, followed by 100 µL of internal standard solution. The mixture was vortexed for 2 minutes and then centrifuged. For the spiked plasma sample preparation, 50 µL of mouse plasma blank and then 50 µL of the L-carnitine and trimethylamine N-oxide working standard were added to a 2 mL centrifuge tube. The solution was mixed well and then 100 µL of internal standard solution were added to the tube. The mixture was vortexed for 2 minutes after securing the cap and then centrifuged. For each sample, the upper supernatant was taken for analysis.

Samples were analyzed using high performance liquid chromatography and mass spectrometry according to the specifications in Tables 2 and 3 below.

**Table 2: High Performance Liquid Chromatography Conditions:**

| | | | |
|---|---|---|---|
| Column | Phenomenex Synergi 4 µ Polar-RP 80A, 150x4.6 mm, 4 µm, part No. 00F-4336-E0 | | |
| Column Temp. | 30 °C | | |
| Sample Temp. | 25 °C | | |
| Injection Vol. | 5 µL | | |
| Flow rate: | 0.5 mL/min | | |
| Mobile Phase A: | 10 mM ammonium formate | | |
| Mobile Phase B: | Methanol | | |

### Gradient:

| Time (min) | | Mobile phase A (%) | Mobile phase B (%) |
|---|---|---|---|
| 0 | | 99 | 1 |
| 5 | | 99 | 1 |
| 9 | | 5 | 95 |
| 10 | | 5 | 95 |
| Post run: 6 min | | | |

**Table 3. Mass Spectroscopy Condition**

| Polarity | Positive |
|---|---|
| MRM Transitions: | 171 → 103.2 |
| | 162 → 60.3 |
| | 85 → 68.3 |
| | 76 → 59.2 |
| Fragmentor | 100 V |
| Capillary Voltaqe | 3000 V |
| Gas Temperature | 350 °C |
| Drying Gas | 10 L/min |
| Nebulizer Pressure | 40 psi |

A standard calibration curve was generated by plotting concentration versus response (the ratio of peak area) of L-carnitine/TMAO to the corresponding internal standards. For each plasma sample, the response of L-carnitine/TAMO to the internal standard in the sample was compared to the standard calibration curve to determine the concentrations of L-carnitine/TMAO. The linear response achieved within the calibration ranges had a calculated correlation coefficient of 0.9945 for L-carnitine and 0.9994 for trimethylamine N-oxide. The limit of detection and limit of quantitation were also determined using general regression. The calculated limit of detection and limit of quantification were, respectively, 0.3 and 0.11 µg/mL for L-carnitine and 0.03 and 0.10 µg/mL for trimethylamine N-oxide. The specificity of the method was also obtained for the analysis of L-carnitine and trimethylamine N-oxide. L-carnitine and trimethylamine N-oxide were completely resolved with respective retention times of 3.8 and 4.4 min. The accuracy of the method was determined from the recovery analysis of samples containing two different levels of L-carnitine and trimethylamine N-oxide. Level 1 contained 1.2 µg/mL of L-carnitine and 0.1 µg/mL of trimethylamine N-oxide, and level 2 contained 3 µg/mL of L-carnitine and 0.25 µg/mL of trimethylamine N-oxide. The recovery of L-carnitine and trimethylamine N-oxide from the spiked samples was 95.6 ± 0.04% (n=5) and 96.4 ± 0.03% (n=5), respectively.

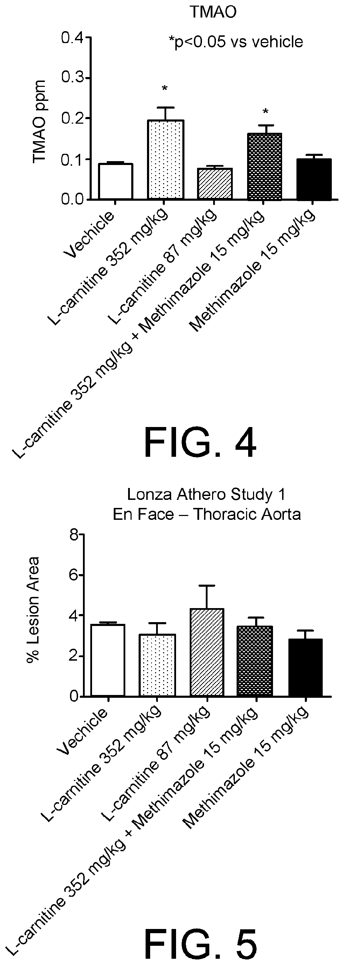

The mice treated with the high dose of L-carnitine (352 mg/kg) and the low dose of L-carnitine (87 mg/kg) showed a significant increase in the concentration of L-carnitine in the plasma compared to the control vehicle (Figure 3). The mice treated with methimazole (15 mg/kg) were shown to have a significant decrease in the concentration of L-carnitine in comparison to the control vehicle (Figure 3). The high dose of L-carnitine (352 mg/kg) treatment and the combination high dose of L-carnitine (352 mg/kg) and methimazole treatment both showed a statistically significant increase in the concentration of TMAO compared to the concentration of TMAO in the vehicle control (Figure 4). Measured TMAO values showed high inter-variability between subjects.

The effect of treatment with L-carnitine and/or methimazole on atherosclerosis progression was determined by measuring the amount of lesions present in the thoracic aorta and the sinotubular junction of the aortic root. After 12 weeks of treatment, the mice were sacrificed, and their tissues were removed for analysis. The En-face thoracic aortas were isolated and prepared for analysis by trimming present fat and fixing the aortas in formalin for 48-72 hours. For morphometric en face analysis, the aortas were laid out, pinned on black matrix, and stained with the lipid binding dye Sudan IV to determine the percentage of the thoracic aorta with lesion present. A Nikon computerized image analysis system was used to image the vessels for surface involvement. En face analysis revealed no significant changes in the amount of plaque present in the thoracic aorta following treatment with L-carnitine and/or methimazole (Figure 5). However, a significant decrease in the percent lesion area of the thoracic aorta was observed at TMAO levels higher than 0.1 ppm versus TMAO levels lower than 0.1 ppm (Figure 6)

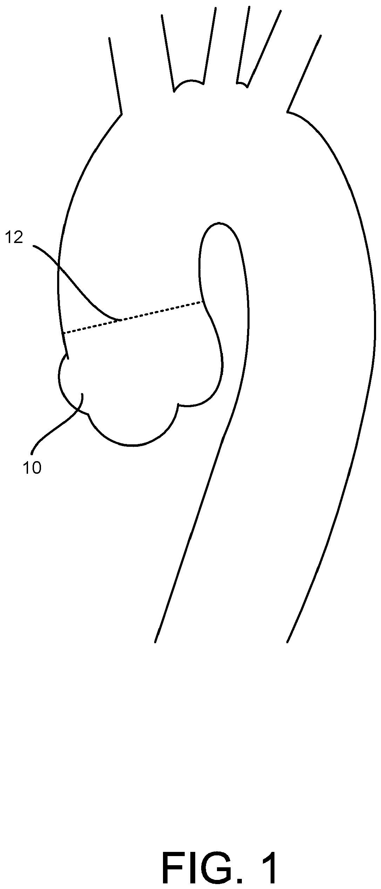

To measure the amount of plaque present in the aortic root, the aortic sinus was prepared for analysis by cutting approximately 5mm of the ascending aorta from the remainder of the aorta, removing the apex of the heart, and placing the remaining heart with the attached aortic segment in OCT medium and freezing it in a dry ice/2-methylbutane bath. Figure 1 illustrates an aortic root 10 positioned below the sinotubular junction 12. Beginning with the ascending aorta, sections of 10 µM in length were cut from the aortic sinus until the ventricular chamber was reached. The aortic sinus sections were then stained with hematoxylin and eosin and imaged at 5 step levels using a Nikon computerized image analysis system. All mice receiving L-carnitine or methimazole treatments showed small but significant reductions in the amount of plaque in the aortic root (Figure 7).

These and other modifications and variations to the present invention may be practiced by those of ordinary skill in the art, without departing from the scope of the present invention, which is set forth in the appended claims.

## Claims

1. A TMAO producing agent for use in a method for treating artherio lesions comprising:
administering to a mammal a TMAO producing agent, the TMAO producing agent comprising a compound that, when ingested or injected, forms a TMAO in the body of the mammal, the TMAO producing agent being administered to the mammal in a pharmaceutically effective amount to increase TMAO levels in the blood of the mammal, wherein the TMAO producing agent comprises a carnitine, a choline, a betaine, or combinations thereof, wherein the carnitine comprises L-carnitine or D-carnitine, and the betaine comprises a betaine salt, a carboxylic derivative of betaine, or a betaine/amino acid complex or compound, or combinations thereof.

2. A TMAO producing agent for use in a method as defined in claim 1, further comprising the step of monitoring levels of TMAO in the blood of the mammal during periodic administration of the TMAO producing agent.

3. A TMAO producing agent for use in a method as defined in claim 2, further comprising the step of adjusting the amount of TMAO producing agent administered to the mammal based upon the levels of TMAO monitored in the blood in order to increase or decrease the amount of TMAO in the blood.

4. A TMAO producing agent for use in a method as defined in claim 1, wherein the TMAO producing agent comprises a mixture of at least two TMAO producing agents selected from the group consisting of a carnitine, a choline, and a betaine.

5. A TMAO producing agent for use in a method as defined in claim 1, wherein the TMAO producing agent comprises a TMAO precursor or a TMAO analog.

6. A TMAO producing agent for use in a method as defined in any of the preceding claims, wherein the TMAO producing agent is ingested by the mammal, or is injected into the mammal.

7. A TMAO producing agent for use in a method as defined in any of the preceding claims, wherein the TMAO producing agent is administered to a mammal afflicted with or at risk of developing atherosclerosis or a disorder associated with or mediated by atherosclerosis, or wherein the TMAO producing agent is administered to a mammal afflicted with a peripheral vascular disease.

8. A TMAO producing agent for use in a method as defined in any of the preceding claims, wherein the TMAO producing agent is administered to the mammal at a dose of greater than 50 mg/kg, such as greater than 70 mg/kg, such as greater than 100 mg/kg, such as greater than 150 mg/kg, such as greater than 200 mg/kg, such as greater than 250 mg/kg, such as greater than 300 mg/kg, such as greater than 350 mg/kg, such as greater than 400 mg/kg.

9. A TMAO producing agent for use in a method as defined in any of the preceding claims, wherein the TMAO producing agent is administered to the mammal once or twice a day.

10. A TMAO producing agent for use in a method as defined in any of the preceding claims, wherein the mammal comprises a human patient or a feline or canine.

## Patentansprüche

1. TMAO erzeugendes Mittel zur Verwendung in einem Verfahren zur Behandlung arterieller Läsionen, umfassend:
Verabreichen eines TMAO erzeugenden Mittels an einen Säuger, wobei das TMAO erzeugende Mittel eine Verbindung umfasst, die, wenn sie eingenommen oder injiziert wird, in dem Körper des Säugers ein TMAO bildet, wobei das TMAO erzeugende Mittel dem Säuger in einer pharmazeutisch effektiven Menge verabreicht wird, um die TMAO-Spiegel im Blut des Säugers zu erhöhen, wobei das TMAO erzeugende Mittel ein Carnitin, ein Cholin, ein Betain oder Kombinationen davon umfasst, wobei das Carnitin L-Carnitin oder D-Carnitin umfasst, und wobei das Betain ein Betainsalz, ein Carboxylderivat von Betain oder einen Betain/Aminosäure-Komplex oder eine Betain/Aminosäure-Verbindung oder Kombinationen davon umfasst.

2. TMAO erzeugendes Mittel zur Verwendung in einem Verfahren wie in Anspruch 1 definiert, des Weiteren umfassend den Schritt des Überwachens der TMAO-Spiegel im Blut des Säugers während periodischer Verabreichung des TMAO erzeugenden Mittels.

3. TMAO erzeugendes Mittel zur Verwendung in einem Verfahren wie in Anspruch 2 definiert, des Weiteren umfassend den Schritt des Anpassens der Menge an TMAO erzeugenden Mittel, die dem Säuger verabreicht wird, basierend auf den im Blut überwachten TMAO-Spiegeln, um die Menge an TMAO in dem Blut zu erhöhen oder abzusenken.

4. TMAO erzeugendes Mittel zur Verwendung in einem Verfahren wie in Anspruch 1 definiert, wobei das TMAO erzeugende Mittel eine Mischung aus mindestens zwei TMAO erzeugenden Mitteln ausgewählt aus der Gruppe bestehend aus einem Carnitin, einem Cholin und einem Betain umfasst.

5. TMAO erzeugendes Mittel zur Verwendung in einem Verfahren wie in Anspruch 1 definiert, wobei das TMAO erzeugende Mittel einen TMAO-Vorläufer oder ein TMAO-Analogon umfasst.

6. TMAO erzeugendes Mittel zur Verwendung in einem Verfahren wie in einem der vorhergehenden Ansprüche definiert, wobei das TMAO erzeugende Mittel von dem Säuger eingenommen wird oder dem Säuger injiziert wird.

7. TMAO erzeugendes Mittel zur Verwendung in einem Verfahren wie in einem der vorhergehenden Ansprüche definiert, wobei das TMAO erzeugende Mittel einem Säuger verabreicht wird, der unter Atherosklerose leidet oder ein Risiko aufweist, diese zu entwickeln, oder unter einer Erkrankung leidet, die mit Atherosklerose assoziert ist oder davon vermittelt wird, oder wobei das TMAO erzeugende Mittel einem Säuger verabreicht wird, der unter einer peripheren Gefäßerkrankung leidet.

8. TMAO erzeugendes Mittel zur Verwendung in einem Verfahren wie in einem der vorhergehenden Ansprüche definiert, wobei das TMAO erzeugende Mittel dem Säuger in einer Dosis von mehr als 50 mg/kg verabreicht wird, wie mehr als 70 mg/kg, wie mehr als 100 mg/kg, wie mehr als 150 mg/kg, wie mehr als 200 mg/kg, wie mehr als 250 mg/kg, wie mehr als 300 mg/kg, wie mehr als 350 mg/kg, wie mehr als 400 mg/kg.

9. TMAO erzeugendes Mittel zur Verwendung in einem Verfahren wie in einem der vorhergehenden Ansprüche definiert, wobei das TMAO erzeugende Mittel dem Säuger ein bis zwei Mal pro Tag verabreicht wird.

10. TMAO erzeugendes Mittel zur Verwendung in einem Verfahren wie in einem der vorhergehenden Ansprüche definiert, wobei der Säuger einen menschlichen Patienten oder einen Feliden oder Kaniden umfasst.

## Revendications

1. Agent de production de TMAO pour une utilisation dans une méthode destinée au traitement de lésions athéroscléreuses, comprenant :
l'administration, à un mammifère, d'un agent de production de TMAO, l'agent de production de TMAO comprenant un composé qui, lors de son ingestion ou son injection, forme un TMAO dans l'organisme du mammifère, l'agent de production de TMAO étant administré au mammifère selon une quantité pharmaceutiquement efficace afin d'augmenter les taux de TMAO dans le sang du mammifère, où l'agent de production de TMAO comprend une carnitine, une choline, une bétaïne, ou des combinaisons de celles-ci, où la carnitine comprend de la L-carnitine ou de la D-carnitine, et la bétaïne comprend un sel de bétaïne, un dérivé carboxylique de bétaïne, ou un complexe ou composé de bétaïne/acide aminé, ou des combinaisons de ceux-ci.

2. Agent de production de TMAO pour une utilisation dans une méthode telle que définie selon la revendication 1, comprenant en outre l'étape consistant à suivre les taux de TMAO dans le sang du mammifère pendant l'administration périodique de l'agent de production de TMAO.

3. Agent de production de TMAO pour une utilisation dans une méthode telle que définie selon la revendication 2, comprenant en outre l'étape consistant à ajuster la quantité d'agent de production de TMAO administré au mammifère sur la base des taux de TMAO suivis dans le sang, afin d'augmenter ou de diminuer la quantité de TMAO dans le sang.

4. Agent de production de TMAO pour une utilisation dans une méthode telle que définie selon la revendication 1, l'agent de production de TMAO comprenant un mélange d'au moins deux agents de production de TMAO choisis dans le groupe constitué par une carnitine, une choline, et une bétaïne.

5. Agent de production de TMAO pour une utilisation dans une méthode telle que définie selon la revendication 1, l'agent de production de TMAO comprenant un précurseur de TMAO ou un analogue de TMAO.

6. Agent de production de TMAO pour une utilisation dans une méthode telle que définie selon l'une quelconque des revendications précédentes, l'agent de production de TMAO étant ingéré par le mammifère, ou étant injecté dans le mammifère.

7. Agent de production de TMAO pour une utilisation dans une méthode telle que définie selon l'une quelconque des revendications précédentes, l'agent de production de TMAO étant administré à un mammifère souffrant, ou risquant de développer, une athérosclérose ou un trouble associé à ou médié par l'athérosclérose, ou l'agent de production de TMAO étant administré à un mammifère souffrant d'une maladie vasculaire périphérique.

8. Agent de production de TMAO pour une utilisation dans une méthode telle que définie selon l'une quelconque des revendications précédentes, l'agent de production de TMAO étant administré au mammifère selon une dose supérieure à 50 mg/kg, telle que supérieure à 70 mg/kg, telle que supérieure à 100 mg/kg, telle que supérieure à 150 mg/kg, telle que supérieure à 200 mg/kg, telle que supérieure à 250 mg/kg, telle que supérieure à 300 mg/kg, telle que supérieure à 350 mg/kg, telle que supérieure à 400 mg/kg.

9. Agent de production de TMAO pour une utilisation dans une méthode telle que définie selon l'une quelconque des revendications précédentes, l'agent de production de TMAO étant administré au mammifère une ou deux fois par jour.

10. Agent de production de TMAO pour une utilisation dans une méthode telle que définie selon l'une quelconque des revendications précédentes, le mammifère comprenant un patient humain ou un animal félin ou canin.
